# EUROPÄISCHE PATENTSCHRIFT

(11) **EP 1 448 214 B1**
(45) Veröffentlichungstag und Bekanntmachung des Hinweises auf die Patenterteilung: **19.05.2010**
(21) Anmeldenummer: 02799726.1
(22) Anmeldetag: 23.11.2002
(51) Int. Cl.: A61K 36/9068, A61K 47/32, A61P 1/08, A61P 1/14, A61P 3/10, A61P 29/00

(54) **INGWER-EXTRAKTZUBEREITUNG**
GINGER EXTRACT PREPARATION
PREPARATION A BASE D'EXTRAIT DE GINGEMBRE

(30) Priorität: 26.11.2001 DE 10159077
(43) Veröffentlichungstag der Anmeldung: 25.08.2004
(73) Patentinhaber: Finzelberg GmbH & Co. KG, 56626 Andernach (DE)
(72) Erfinder: GAEDCKE, Frauke, 56323 Waldesch (DE); FEISTEL, Björn, 56626 Andernach (DE)
(74) Vertreter: Wenzel & Kalkoff
(86) Internationale Anmeldenummer: PCT/EP2002/013148
(87) Internationale Veröffentlichungsnummer: WO 2003/045411

(56) Entgegenhaltungen:
- WO-A-02/41838
- DE-A- 19 859 499
- PATENT ABSTRACTS OF JAPAN vol. 1997, no. 06, 30. Juni 1997 (1997-06-30) & JP 09 052844 A (AMANO PHARMACEUT CO LTD), 25. Februar 1997 (1997-02-25)
- DATABASE MEDLINE [Online] August 1992 (1992-08) LI S ET AL: "[Stability examination of beta-cyclodextrin inclusion compound of Zingiber officinale volatile oil]" Database accession no. NLM1482534 XP002240213 & ZHONGGUO ZHONG YAO ZA ZHI = ZHONGGUO ZHONGYAO ZAZHI = CHINA JOURNAL OF CHINESE MATERIA MEDICA. CHINA AUG 1992, Bd. 17, Nr. 8, August 1992 (1992-08), Seiten 481-483, 511, ISSN: 1001-5302

## Beschreibung

Die Erfindung betrifft Ingwer-Extraktzubereitungen, die über einen längeren Zeitraum, d.h. über einen Zeitraum von bis zu 18 Monaten und mehr, die Merkmale einer stabilen Extraktzubereitung erfüllen, sowie ein Verfahren zu deren Herstellung. Die Erfindung beschreibt weiterhin stabile Ingwer-Extraktzubereitungen enthaltende galenische Zubereitungen wie Kapseln, Dragees und Tabletten und deren Verwendung.

Ingwer (Zingiber officinale), dessen Eigenschaft als Heil- und Gewürzpflanze bereits seit Jahrhunderten bekannt ist, hat in den letzten Jahren in der pharmazeutischen Industrie und im Nahrungsergänzungsmittelbereich zunehmend an Bedeutung gewonnen. Zubereitungen aus dem Wurzelstock des Ingwers (Rhizoma Zingiberis) wie dem Drogenpulver selbst und daraus gewonnenen Extrakten werden zur Behandlung dyspeptischer Beschwerden sowie der Symptome der Reisekrankheit eingesetzt (vgl. hierzu die Literaturzusammenfassung in Langner, E. et al.: Balance 1, 5-16, Oktober 1997). Der Vielzahl pharmakologischer Untersuchungen trug auch die Kommission E des früheren Bundesgesundheitsamtes Rechnung, indem sie eine positive Monographie "Zingiberis rhizoma" veröffentlicht hat (Bundesanzeiger Nr. 85 vom 05.05.1988 sowie Bundesanzeiger Nr. 50 vom 13.03.1990). Die mittlere Tagesdosis beträgt 2-4 g Droge. Als für die Wirksamkeit mitverantwortliche Inhaltsstoffe werden das ätherische Öl sowie insbesondere die Scharfstoffe (Gingerole, Shogaole und Dehydrogingerdione) angesehen. Marktübliche, monographiekonforme Zubereitungen sind derzeit Drogenpulver und Tee-schnitte mit einer monographiekonformen Dosierung von 2-4 g sowie entsprechende Extrakte, die mit Alkohol/Wasser-Gemischen hergestellt werden . Mit überkritischem CO₂ hergestellte Extrakte werden dagegen von der Monographie der Kommission E wegen nicht ausreichend belegter Wirksamkeit und Unbedenklichkeit nicht abgedeckt. Diese sind in der Lebensmittelindustrie jedoch als Gewürzextrakte üblich.

Innerhalb der Scharfstoffe bilden die Gingerole den mengenmäßigen Hauptanteil. Shogaole und Dehydrogingerdione kommen in deutlich geringen Mengen vor, da sie biogenetisch nur Nebenprodukte der Gingerole darstellen (Schuhbaum,H., Franz,G.: Zeitschrift für Phytotherapie 21, 203-209, 2000).

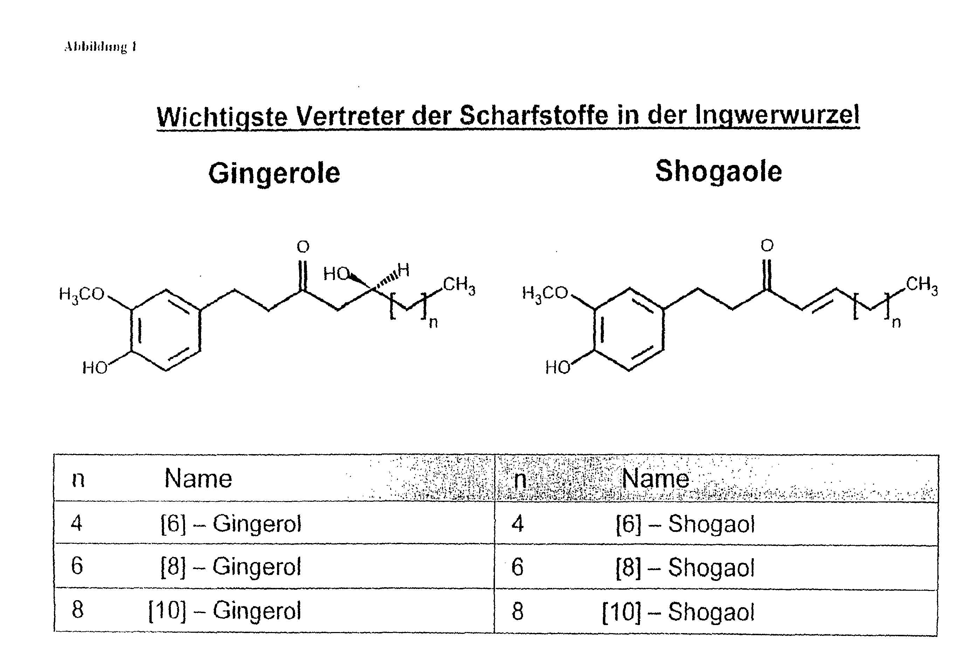

Die Gingerole besitzen verschieden lange Seitenketten (6-, 8- und 10-C-Atome, s. Abbildung 1), wobei das [6]-Gingerol die Hauptkomponente darstellt (Falch,B. Reichling,J., Saller,R.: Dtsch. Apotheker Zeitung 137, 47-60). Neben den Gingerolen sind auch für das [6]-Shogaol pharmakologische Effekte beschrieben (Suekawa,M. et al.: J. Pharm. Dyn. 7, 836-848, 1984).

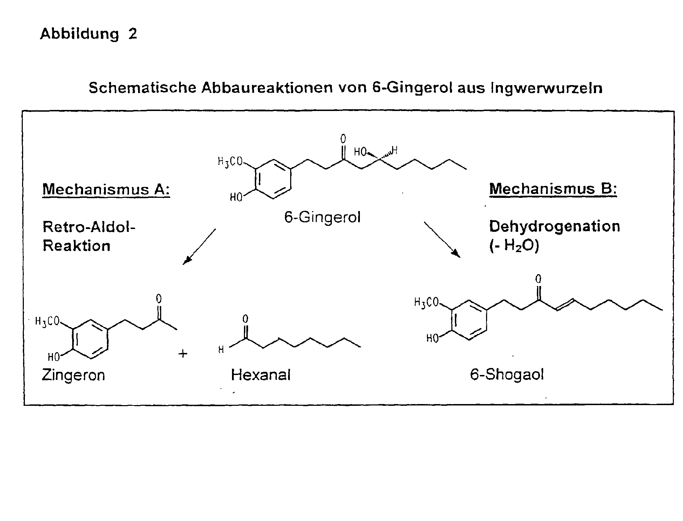

Es ist bekannt, daß Shogaole während der Lagerung von ganzen und besonders von zerkleinerten Ingwerwurzeln aus den Gingerolen entstehen (Zhang,X. et al.: J. Food Science, 59, 1338-1343, 1994). Auch für gepulverte Ingwerwurzeln ist bei Lagerung eine stetige Umwandlung der Gingerole zu Shogaolen beschrieben (Steinegger,E., Stucki,K.: Pharm. Acta. Helv. 57, 3, 1982) s. Abbildung 2 - Mechanismus A.

Ferner ist beschrieben, daß der Gehalt an Gingerolen in alkoholisch-wäßrig hergestellten Extrakten zugunsten der Bildung von Shogaolen abnimmt (Dissertation Germer S., Universität Regensburg 1996); s. Abbildung 2 - Mechanismus B. Das Verhältnis von [6]-Gingerol zu [6]-Shogaol stellt somit einen qualitätsrelevanten Parameter zur Beurteilung der Herstellung von Ingwer-Extrakten und deren Weiterverarbeitung dar (Feistel, B., Gaedcke, F. et al.: Analytische Charakterisierung von Ingwer-Zubereitungen, Poster-Nr. P19, Finzelberg-Symposium 2000).

Um eine gleichbleibende, therapeutische Qualität von Ingwerzubereitungen zu gewährleisten, muß eine reproduzierbare Qualität sichergestellt sein. Grundvoraussetzung hierfür ist, daß die Umwandlung von Gingerolen zu Shogaolen möglichst unterbunden wird (konstantes Verhälnis von [6]-Gingerol zu [6]-Shogaol).

Es ist daher wünschenswert, Ingwer-Präparate zur Verfügung zu stellen, bei denen die bekannte Umlagerungsreaktion der Gingerole vermieden und ein möglichst gleichbleibendes Gingerol/Shogaol-Verhältnis sichergestellt wird. Als stabil im pharmazeutischen Sinne gelten bei pharmazeutisch relevanten Inhaltsstoffen solche Präparate, bei denen die Schwankungen eines Inhaltsstoffes über die Laufzeit nicht mehr als bis zu +/- 10 % betragen.

Die JP 09 052844 offenbart eine Enzymlösung, die PVP enthält sowie darüber hinaus Ingwerextrakt enthalten kann. Dabei ist das PVP in der Lösung enthalten, um das Enzym zu stabilisieren und dessen Ausfällung zu verhindern.

Die EP 0 826 372 offenbart, wie in das in einer Pflanze vorliegende Gemisch an Inhaltsstoffen, das genuine Substanzgemisch, möglichst vollständig in den entsprechenden Extrakt überführt werden kann, so dass dieser in seiner Zusammensetzung der Pflanze selbst möglichst nahe kommt. Diese Schrift betrifft somit die Gewinnung eines Pflanzenextraktes. Dabei ist die Form des Extraktes unerheblich. Es kann sich um einen Flüssig-, Spissum- oder Trockenextrakt handeln.

Aus der DE 198 59 499 A1 ist bekannt, daß Ingwerextrakte durch Zusatz von mindestens einem galenischen Hilfsstoff stabilisiert werden können, so daß der Scharfstoffgehalt (hier lediglich die Summe der Hauptsubstanz 6-Gingerol und seines Abbauproduktes 6-Shogaol) über einen Zeitraum von 18 Monaten maximal um 10 % abnimmt. Als Hilfsstoffe werden dabei Öle, halbfeste Triglyceride, Fettsäuren und Fettalkohole genannt. Auf diese Weise gelingt es, daß 6-Gingerol nur um bis zu 20 % abnimmt, während 6-Gingerol in einem nativen Ingwerextrakt unter gleichen Bedingungen bis ca. 32 % abbaut.

Der Parameter Scharfstoffgehalt (angegeben als Summe an 6-Gingerol und dessen Abbauprodukt 6-Shogaol) bleibt dabei naturgemäß konstant (siehe Umwandlung gem. Abbildung 2), so daß er für eine Qualitätsaussage nicht geeignet ist. Aussagekräftig ist hier nur das Verhältnis von 6-Gingerol zu 6-Shogaol in Verbindung mit dem Scharfstoffgehalt (Summe von 6-, 8-, und 10-Gingerol sowie 6-Shogaol).

Die in Patentschrift DE 198 59 499 A1 angeführten lipophilen Hilfsstoffe bewirken, daß die Gingerole auf physikalischem Wege durch Erhöhung der Viskosität stabilisiert werden. Nachteilig ist jedoch, daß die hier verwendeten galenischen Hilfsstoffe (Öle, Fette) nicht in der Lage sind, die dem Stabilitätsverlust der Gingerole zugrunde liegende Dehydratisierung zu verhindern. Dies beinhaltet auch, daß gemäß diesem Verfahren ölige, pastöse Zubereitungen entstehen, die in der Regel nur in Weichgelatinekapseln abgefüllt werden können, wie dies bisher auch für lipophile Ingwer-Spissa oder -Oleosa bekannt ist. Stabile, feste galenische Darreichungsformen von öligen Ingwer-Extrakten wie Tabletten, Kapseln oder Dragees sind bisher nicht bekannt.

Eine Aufgabe der vorliegenden Erfindung ist es daher, Ingwer-Extraktzubereitungen zur Verfügung zu stellen, die eine langfristige Stabilität der Gingerole sowie des Gingerol/Shogaol-Verhältnisses sicherstellen und die darüber hinaus auf einfache Weise zu verschiedenen festen, stabilen Darreichungsformen verarbeitet werden können.

Diese Aufgabe wird erfindungsgemäß durch die Herstellung von Ingwer-Extraktzubereitungen gelöst, umfassend einen Gehalt an Ingwerextrakt, die mindestens einen stabilisierenden Hilfsstoff aus der Gruppe der Polyvinyl-pyrrolidone. Überraschenderweise wurde gefunden, daß durch den Zusatz solcher Substanzen die Abbaureaktionen der Gingerole weitestgehend unterbunden, so daß Ingwer-Extraktzubereitungen erhalten werden, die über viele Monate einen im wesentlichen stabilen [6]-Gingerol-Gehalt sowie ein gleichbleibendes Verhältnis von [6]-Gingerol zu [6]-Shogaol aufweisen. Die Protonierung der Ingwer-Inhaltsstoffe, die eine Dehydratisierung und damit eine Zersetzung dieser Stoffe bewirkt, wird dadurch verhindert, daß das Polyvinylpyrrolidon als protonenabfangende Substanz die vorhandenen freien Protonen aufnimmt bzw. einen Angriff von vorhandenen freien Protonen abwehrt.

Die erfindungsgemäßen Ingwer-Extraktzubereitungen sind nicht ölig, wie man dies von den bisherigen Zubereitungen kennt. Vielmehr handelt es sich um trockene, rieselfähige Extraktzubereitungen, die auch direkt tablettierbar sind. Weiterhin ist die Temperaturanfälligkeit der Gingerole gegenüber marktüblichen Ingwer-Extraktzubereitungen stark verringert. So ergab ein Streßtest von erfindungsgemäßen Ingwer-Extraktzubereitungen bei 40°C keinerlei Veränderung im Gingerol-Gehalt (siehe beigefügte Tabelle ).

**Tabelle 1: Vergleichende Stabilität von Ingwer-Extraktzubereitungen über 4 Wochen bei 40°C. (In Klammern ist das jeweils vorliegende Verhältnis 6-Gingerol zu 6-Shogaol als Maß für die Stabilität angegeben.)**

| | Erfindung Muster 9101.1100 | Stand der Technik (DE 198 59 499 A1) |
|---|---|---|
| Ausgangswert 6-Gingerol | 2,29 % (2,6 : 1) | 8,11 % (2,0 : 1) |
| 4 Wochen 40°C | 2,23 % (2,6 : 1) | 6,80 % (1,3 : 1) |

Die Verwendung von Polyvinylpyrrolidonen (Kollidone, polymere N-Vinylpyrrolidone) als Hilfsstoff für die Herstellung von galenischen Formen ist seit vielen Jahren bekannt. Sie werden als galenisches Sprengmittel eingesetzt und dienen insbesondere dazu, die Löslichkeit von schwerlöslichen Arzneistoffen zu erhöhen. Auch bei Pflanzenextrakten werden Polyvinylpyrrolidone verwendet. So ist es aus der WO99/32130 bekannt, Polyvinylpyrrolidone einzusetzen, um die Freisetzung wertgebender Inhaltsstoffe von Arzneipflanzentrockenextrakten zu verbessern. Hierzu verwendet man einen halbfesten oder festen Komplex aus Pflanzenextrakt und Träger, bei dem die wertgebenden Extraktbestandteile mikrodispers so verteilt vorliegen, daß deren Freisetzung sowohl im Grad als auch in der Geschwindigkeit auf hohem Niveau standardisierbar sind. Die neben anderen Stoffen wie Polyethylenglycolen, Polyvidonacetaten und Polyvinylglycolen angeführten Polyvinylpyrrolidone, Cellulose- oder Stärkederivate bewirken eine verbesserte Wasserlöslichkeit und durch die Oberflächenvergrößerung,-eine ebenfalls verbesserte Freisetzung der Inhaltsstoffe. Wie auch in anderen Fällen, macht man sich bei dem Einsatz gemäß der WO99/32130 damit den Einfluß der Polyvinylpyrrolidone auf die physikalischen Eigenschaften der Arzneipflanzen-trockenextrakte zunutze.

Überraschenderweise hat sich im Rahmen der vorliegenden Erfindung gezeigt, daß im Falle von Ingwer-Extraktzubereitungen Polyvinylpyrrolidone darüber hinaus auch chemisch Einfluß nehmen und stabilisierend auf die Inhaltsstoffe wirken. Indem sie als Protonenfänger wirken, verhindern sie die Protonierung und die daraus folgende Dehydratisierung der Gingerole. Dieses chemische Prinzip ist während des Herstellungsprozesses der Ingwer-Extraktzubereitungen (in flüssigem Medium) wie auch bei der langfristigen Lagerung (in fester Form) aktiv.

Zu der Gruppe von protonenabfangenden Substanzen zählen insbesondere alle pharmazeutischen Hilfsstoffe mit Stickstoffverbindungen aufgrund des freien Elektronenpaares. Protonenabfangend können auch Substanzen wie Zeolithe oder cyclische Oligosaccharide wirken, in denen durch Bildung eines Extrakt-Hilfsstoff-Einschlußkomplexes die Reaktionen von Protonen mit den Gingerolen verhindern. Diese Hilfsstoffe haben sich zwar als qualitativ geeignet zur Stabilisierung von Ingwer-Extraktzubereitungen herausgestelllt, jedoch gewährleisten sie nicht gleichzeitig die quantitative Verfügbarkeit der Ingwer-Scharfstoffe, wie es z.B. die Polyvinylpyrrolidone vermögen.

Insbesondere von den Cyclodextrinen, z.B. β-Cyclodextrin, ist bekannt, daß sie in der Lage sind, die zu stabilisierenden Ingwer-Inhaltsstoffe räumlich von der Extraktmatrix abzutrennen und so deren Protonierung zu verhindern. Dieses Prinzip wird z.B. zur Maskierung des scharfen Ingwergeschmackes z.B. in Kautabletten angewandt (vgl. DE-Gbm. 201 02 817). Dieser Einschlußkomplex ist jedoch nicht in der Lage, die Scharfstoffe anschließend wieder quantitativ freizusetzen (teilweise irreversible Einschlußkomplexe). Dieser Hilfsstoff ist daher nicht für die arzneiliche Verwendung von Ingwer-Extraktzubereitungen geeignet.

Zur Sicherstellung der Stabilisierung ist das Mengenverhältnis protonenabfangender Hilfsstoff zu nativem Extrakt vorzugsweise größer als 50 %, insbesondere liegt es zwischen 60 und 90 %, wobei sich ein Wert von etwa 75 % als besonders vorteilhaft erwiesen hat.

Als nachteilig haben sich die bisher bekannten Verfahren zur Herstellung von Ingwer-Extraktzubereitungen auch deshalb herausgestellt, weil bei diesen pharmazeutisch relevante Inhaltsstoffe wie die Scharfstoffe und das aetherische Öl im resultierenden Extrakt vermindert sind.

Eine weitere Aufgabe der vorliegenden Erfindung ist es daher, ein Verfahren zur Herstellung stabiler Ingwer-Extraktzubereitungen zur Verfügung zu stellen, bei dem darüber hinaus gewährleistet wird, daß eine größtmögliche Menge an pharmazeutisch relevanten Substanzen in die Extraktzubereitung überführt wird.

Diese Aufgabe wird erfindungsgemäß durch ein Verfahren gelöst, bei dem die Verfahrensschritte bei einer Temperatur von maximal 45°C durchgeführt werden. Bei diesen schonenden Temperaturen werden die Prozesse, die zu einer Verminderung der Ingwer-Inhaltsstoffe führen, reduziert und so Ingwer-Extraktzubereitungen mit einem deutlich erhöhten Anteil pharmazeutisch relevanter Inhaltsstoffe erhalten. Als besonders bevorzugter Temperaturbereich hat sich der Bereich zwischen 35 und 45°C herausgestellt, da diese Temperaturen einerseits hoch genug sind, um eine ausreichende Geschwindigkeit des Extraktionsprozesses zu gewährleisten, andererseits aber gering genug sind, um eine schonende Prozeßführung sicherzustellen.

Durch eine derart schonende Prozeßführung wird sichergestellt, daß das Verhältnis von [6]-Gingerol zu [6]-Shogaol, das, wie eingangs erwähnt, einen qualitätsrelevanten Parameter zur Beurteilung der Herstellung von Ingwer-Extrakten und deren Weiterverarbeitung darstellt, deutlich höher und stabiler ist als bei herkömmlichen Herstellungsverfahren.

Vorzugsweise wird bei dem Herstellungsverfahren mindestens ein stabilisierender Hilfsstoff aus der Gruppe der Polyvinylpyrrolidone verwendet. Die Verwendung mindestens einer Substanz aus der Gruppe der protonenabfangenden Substanzen stellt sicher, daß die durch Protonierung bedingten Zersetzungsreaktionen der Ingwer-Inhaltsstoffe minimiert werden.

Die zur Extraktion des Ingwerwurzelstockes eingesetzten Auszugsmittel gehören vorzugsweise aus der Gruppe der Alkohole mit C1-C4 Kohlenstoffatomen, aus der Gruppe der aliphatischen Ketone mit C1-C5 Kohlenstoffatomen, aus der Gruppe aliphatischer Kohlenwasserstoffe, aus der Gruppe wässrig-alkoholischer Lösungsmittelgemische von 1-99 % V/V , aus der Gruppe von Lösungsmittelgemischen aus Wasser und Ketonen von 1-99 % V/V bzw. sind reines Wasser oder ein -überkritisches Gas wie Kohlendioxid.

Die Primärextraktion von Rhizoma Zingiberis mit einem vorzugsweise polaren Auszugsmittelgemisch sichert durch schonende, erschöpfende Perkolation eine größtmögliche Ausbeute an pharmazeutisch, relevanten Inhaltsstoffen, wie z.B. die Scharfstoffe oder das ätherische Öl. Hierzu wird die Droge mit einem 10- bis 12-fachen Überschuß an Lösemitteln bei einer Temp. von max. 45°C eingesetzt. Die erhaltenen Eluate werden vereinigt und schonend im Vakuum eingeengt. Die erhaltenen pastösen Extrakte werden mit Ethanol auf eine Trockensubstanz von ca. 20 % eingestellt. Die verdünnten ethanolischen Lösungen werden mit den ebenfalls rein ethanolischen Lösungen von stabilisierenden Hilfsstoffen mit einem Trockensubstanzanteil von ca. 10 % bei Raumtemperatur durch Rühren gut gemischt. Anschließend erfolgt eine schonende Aufkonzentrierung dieser Mischung im Vakuum bei 35-45°C Produkttemperatur. Diese schonende Herstellung gewährleistet den Erhalt eines pastösen Extraktes ohne verminderten Gehalt an Scharfstoffen und/oder ätherischem Öl. Die abschließende Trocknung erfolgt wiederum schonend im Vakuumtrockenschrank bei 35-45°C unter Zusätzen von Trockenhilfsstoffen wie z.B. Maltodextrinen oder von Siliziumdioxiden. Es entsteht eine trockene, rieselfähige und stabile Ingwer-Extraktzubereitung.

Vorzugsweise umfaßt das Verfahren zur Herstellung die Verfahrensschritte
- schonende Primärextraktion der zerkleinerten Ingwerwurzeln,
- schonende weitere Konzentrierung, insbesondere durch Extraktion mit überkritischem Kohlendioxid,
- Zugabe mindestens eines stabilisierenden Hilfsstoffes zu der flüssigen Extraktlösung mit 20% Trockensubstanz,
- abschließende schonende Eindampfung und Trocknung der so erhaltenen Extraktzubereitung.

Eine weitere, schonende Konzentrierung und Trocknung hat den Vorteil, daß die resultierenden Ingwer-Extraktzubereitungen ein rieselfähiges Pulver darstellen, das zugleich praktisch frei von Aflatoxin-Verunreinigungen ist.

Als besonders vorteilhafte Form der Konzentrierung hat sich dabei die Extraktion mit überkritischem Kohlendioxid herausgestellt, wobei auch andere Verfahren denkbar und möglich sind, wie beispielsweise säulenchromatographische oder Flüssig-Flüssig-Verfahren.

Schließlich ist es Aufgabe der Erfindung, galenische Zubereitungen zur Verfügung zu stellen, bei denen dadurch, daß diese stabile Ingwer-Extraktzubereitungen enthalten, auch über einen längeren Zeitraum und bei unterschiedlichen Chargen eine gleichbleibende Qualität sichergestellt werden kann.

Diese Aufgabe wird durch galenische Zubereitungen gelöst, die die erfindungsgemäßen Ingwer-Extraktzubereitungen enthalten, wobei die Zubereitungen zusätzlich übliche Hilfsstoffe wie Siliziumdioxide, Maltodextrine, Magnesiumstearate, Cellulosen oder Carboxymethlystärken enthalten können. Vorzugsweise sind solche galenischen Zubereitungen für die Verarbeitung in jegliche Formen von Kapseln, Tabletten und Dragees vorgesehen.

Durch die Erfindung wird es erstmals möglich, eine trockene, stabilisierte Ingwer-Extraktzubereitung zu erhalten. Aus DE 198 59 499 geht hervor, daß ein ethanolischwässrig hergestellter Extrakt, der mittels adsorbierender galenischer Hilfsstoffe (Siliziumdioxid) in eine trockene Ingwer-Extraktzubereitung überführt wird, bereits nach 2 Monaten eine Abnahme von 6-Gingerol um ca. 37 % aufweist. Entsprechend ist es erst durch diese Erfindung möglich, Ingwer-Extraktzubereitungen ohne Stabilitätsverlust in Tabletten zu verpressen. Da durch die vorliegende Erfindung darüber hinaus die Stabilität von Ingwer-Extraktzubereitungen signifikant erhöht wird, kann auch eine wesentlich längere Haltbarkeit als bei nicht stabilisierten, galenischen Zubereitungen garantiert werden.

Die erfindungsgemäße Ingwer-Extraktzubereitung kann insbesondere als Arzneimittel zur Behandlung von dyspeptischen Beschwerden, von Symptomen der Reisekrankheit, als Antiemetikum, als Antidiabetikum, als Analgetikum, bei schwangerschaftsbedingtem oder chemotherapeutisch induziertem Erbrechen, bei Arteriosklerose, bei Erkrankungen des rheumatischen Formenkreises oder als entsprechendes Nahrungsergänzungsmittel verwendet werden.

Weitere Vorteile und Ausgestaltungen gehen aus den nachfolgend beschriebenen Beispielen und Versuchen hervor. Zur Veranschaulichung sind des weiteren zwei Abbildungen beigefügt, von denen die Abbildung 1 die wichtigsten Vertreter der Scharfstoffe in der Ingwerwurzel und die Abbildung 2 die Abbaureaktionen am Beispiel der Hauptscharfstoffverbindung 6-Gingerol wiedergibt.

**Vergleichsbeispiel 1 - Probe A:** Nicht stabilisierter Ingwer-Spissumextrakt (Extr. Zingiberis e rhiz. spir. spiss.)

50 kg Rhizoma Zingiberis, geschnitten und gesiebt zu Teilstückchen von 6-8 mm, werden mit 500 kg Ethanol 96 % [V/V] erschöpfend perkoliert. Die Produkttemperatur überschreitet dabei 45°C nicht. Die filtrierten, ethanolischen Abläufe werden vereinigt und schonend im Vakuum bei 45°C zu pastösen Extrakten eingeengt.

### Beispiel 2- Probe B: Ingwerzubereitung, stabilisiert mit Kollidon 25

45,5 g Ausgangsextrakt - hergestellt wie Probe A (Extractum Zingiberis e rhiz. spir. spiss. mit einem Trockensubstanzgehalt von 33 %) - entspr. 15 g Trockenextrakt - werden mit Ethanol > 99% auf 20 % Trockensubstanz verdünnt. Es wird 15 Minuten lang intensiv gerührt - Lösung 1.

99,75 g Kollidon 25 werden unter Rühren mit Ethanol > 99 % auf 10 % Trockensubstanz verdünnt. Es wird 30 Minuten lang intensiv gerührt - Lösung 2.

Beide Lösungen werden unter Rühren bei Raumtemperatur portionsweise vereinigt, wobei eine rotbraune, klare Lösung entsteht. Es wird 30 Minuten lang gerührt und am Rotationsverdampfer schonend bei maximal 45°C Wasserbadtemperatur eingeengt. Die erhaltene Extraktzubereitung wird lösungsmittelfrei eingedampft und im Trockenschrank bei 45°C unter Vakuum getrocknet. Als Hilfsstoff wird 5 % Siliziumdioxid zugesetzt.

Bei den Proben A und B wurden zu Beginn (= Startwerte) sowie nach 11 und 15 Monaten jeweils die Scharfstoffgehalte (6-,8-,10-Gingerol und 6-Shogaol) mit einem HPLC-Verfahren überprüft. Dabei wurden die in der folgenden Tabelle 2 wiedergegebenen Daten erhalten:

**Tabelle 2: Stabilität von erfindungsgemäßen Zubereitungen gegenüber dem nicht stabilisierten Ausgangsextrakt A. Der Scharfstoffgehalt der Extraktzubereitungen B und C wurde zur Vergleichbarkeit auf den nativen Extrakt berechnet. (In Klammern ist das Verhältnis von 6-Gingerol zu 6-Shogaol als Maß für den Grad der erfolgten Umlagerung angegeben).**

| Probe | Analytische Parameter | Beginn (Startwert) | 11 Monate | 15 Monate |
|---|---|---|---|---|
| A | Scharfstoffgehalt | 20 % | 15,9% | 15,4% |
| | Verhältnis 6-Gingerol : 6-Shogaol | (3,4:1) | (1:1) | (0,7:1) |
| B | Scharfstoffgehalt | 15,6 % | 15,6 % | 15,6 % |
| | Verhältnis 6-Gingerol : 6-Shogaol | (1,9:1) | (1,9:1) | (1,9:1) |

Es zeigt sich, daß bei dem nicht stabilisierten Extrakt A der Scharfstoffgehalt um fast 25 % abnimmt und sich insbesondere auch das Verhältnis 6-Gingerol zu 6-Shogaol umkehrt; dagegen weisen die erfindungsgemäß stabilisierten Extraktzubereitung B eine deutlich verbesserte Stabilität betreffend den Scharfstoffgehalt auf als auch insbesondere ein innerhalb der analytischen Fehlertoleranz konstantes Verhältnis von 6-Gingerol zu 6-Shogaol.

Des weiteren wurden Stabilitätsprüfungen an Proben mit unterschiedlichem nativen Extraktanteil mit folgenden Ergebnissen durchgeführt (Tabelle 3).

**Tabelle 3: Stabilitätsdaten von Ingwer-Extraktzubereitungen mit Kollidon 25 als Hilfsstoff (bei Raumtemperatur)**

| | | Zeit [Monate] | | | |
|---|---|---|---|---|---|
| 13% nativer Extraktanteil | Start | 6 | 12 | 18 | Veränderungen |
| **Scharfstoffgehalt [%]** | **3,14** | **3,12** | **3,16** | **3,09** | **- 1,9%*** |
| 6-Gingerol:6-Shogaol | 4,9 : 1 | 4,5 : 1 | 4,5 : 1 | 4,6 : 1 | konstant* |
| 6-Gingerol [%] | 1,82 | 1,78 | 1,78 | 1,79 | -1,7 %* |
| 6-Shogaol [%] | 0,37 | 0,40 | 0,40 | 0,38 | +2,5 %* |
| | | | | | |

| | | Zeit [Monate] | | | |
|---|---|---|---|---|---|
| 30% nativer Extraktanteil | Start | 2 | 8 | 16 | Veränderungen |
| **Scharfstoffgehalt [%]** | **4,34** | **4,29** | **4,19** | **4,18** | **- 3,7 %** |
| 6-Gingerol:6-Shogaol | 2,6:1 | 2,6:1 1 | 2,6:1 1 | 2,6:1 | stabil |
| 6-Gingerol [%] | 2,29 | 2,16 | 2,12 | 2,18 | -4,8% |
| 6-Shogaol [%] | 0,88 | 0,83 | 0,82 | 0,81 | -8,0 % |
| | | | | | |

| | | Zeit [Monate] | | | |
|---|---|---|---|---|---|
| 49% nativer Extraktanteil | Start | 2 | 8 | 16 | Veränderungen |
| **Scharfstoffgehalt [%]** | **9,00** | **8,80** | **8,77** | **8,74** | **- 2,9%** |
| 6-Gingerol:6-Shogaol | 2,7 : 1 | 2,8 : 1 | 2,8 : 1 | 2,9 : 1 | stabil |
| 6-Gingerol [%] | 4,77 | 4,68 | 4,58 | 4,67 | -2,1 % |
| 6-Shogaol [%] | 1,78 | 1,67 | 1,61 | 1,66 | -6,7% |

| | | | | | |
|---|---|---|---|---|---|
| * Die relativen Veränderungen einiger Gehalte liegen teilweise innerhalb des analytischen Fehlerbereiches und sind daher marginal. | | | | | |

Es zeigt sich, daß die Ingwer-Extraktzubereitungen auch über einen langen Zeitraum stabil bleiben. Im Gegensatz dazu ergibt sich bei einem unbehandelten Ingwerextrakt das in Tabelle 4 dargestellte Verhalten:

**Tabelle 4: Stabilitätsdaten eines nicht stabilisierten Ingwerextraktes; eine Weiterverfolgung über 8 Wochen hinaus war angesichts der Ergebnisse nicht sinnvoll.**

| | | Zeit [Wochen] | | | | |
|---|---|---|---|---|---|---|
| Ausgangsextrakt | Start | | 8 | | | Veränderungen |
| **Scharfstoffgehalt [%]** | **11,2** | | **9,0** | | | **- ca. 20 %** |
| Verh. 6-Gingerol:6-Shogaol | 3,1 : 1 | | 0,8 : 1 | | | Umkehr des Verhältnisses; Umlagerung |

Stabilisierte Ingwer-Extraktzubereitungen mit verschiedenen nativen Extraktanteilen wurden außerdem auf ihre Temperaturstabilität über 6 Monate untersucht. Das Ergebnis ist in der Tabelle 5 wiedergegeben:

**Tabelle 5: Ergebnisse des Scharfstoffgehaltes während der Streßprüfung von stabilisierten Ingwer-Extraktzubereitungen über 6 Monate. (In Klammern ist das Verhältnis von 6-Gingerol zu 6-Shogaol als Maß für den Grad der erfolgten Umlagerung angegeben).**

| **Probenbezeichnung** | **Scharfstoffgehalt (Verhältnis 6-Gingerol : 6-Shogaol)** | | | **Aussehen nach 6 Monaten** |
|---|---|---|---|---|
| **Temperatur** | **Start-wert** | **2 Wochen-Wert** | **6 Monats-Wert** | |
| **30 % nativer Extrakt (Spir. Spiss.)** | | | | |
| **25°C / 60% rF** | 4,34 (2,6 : 1) | 4,26 (2,6 : 1) | 4,19 (2,6: 1) | pulvrig |
| **30°C / 60 % rF** | | 4,23 (2,6 : 1) | 4,17 (2,5 : 1) | pulvrig |
| **40°C / 75 % rF** | | 4,18 (2,4 : 1) | 4,16 (2,2 : 1) | leicht klumpig |
| **49 % nativ Extrakt (Spir. Spiss.)** | | | | |
| **25°C / 60 % rF** | 9,00 (2,7 : 1) | 8,59 (2,7 1) | 8,70 (2,8 : 1) | klumpig |
| **30°C / 60 % rF** | | 8,44 (2,6 : 1) | 8,61 (2,7: 1) | gesintert |
| **40°C** / **75** % **rF** | | 8,61 (2,7: 1) | 8,24 (2,3 : 1) | gesintert |
| **23 % nativer Extrakt (Oleosum*)** | | | | |
| **25°C / 60 % rF** | 4,90 (6,1 : 1) | 4,94 (5,9 : 1) | 4,94 (5,8: 1) | pulvrig |
| **30°C / 60 % rF** | | 4,95 (5,8 : 1) | 5,05 (5,7 : 1) | pulvrig |
| **40°C / 75 % rF** | | 4,81 (5,6 : 1) | 4,91 (4,6 :1) | leicht klumpig |

| | | | | |
|---|---|---|---|---|
| * hergestellt durch Extraktion mit überkritischem Kohlendioxid | | | | |

Stabilitätsversänderungen innerhalb einer Spanne von +/-5 % bis +/- 10 % gelten bei pharmazeutisch relevanten Inhaltsstoffen im pharmazeutischen Sinne als stabil. Unabhängig von der eigentlichen Prüfung bei erhöhter Temperatur und Feuchtigkeit gemäß den ICH-Richtlinien wurde festgestellt, daß bereits optisch die Zubereitung mit einem erhöhten nativen Extraktanteil von 49 % zu Verklumpungen neigt. Dieser Eindruck wurde durch die Temperaturerhöhung noch verstärkt. Diese Extraktzubereitung eignet sich daher nicht für die Betrachtung der Langzeit-Streßprüfung. Der Gehalt der Scharfstoffe blieb bei den zwei anderen Ingwer-Extraktzubereitungen, auch bei erhöhten Temperaturen, bis zum 6-Monatswert innerhalb der +/-5%-Grenze. Die erfolgreiche Stabilisierung ist somit nachgewiesen.

In der folgenden Tabelle 6 ist die thermische Stabilität eines unbehandelten Extraktes und einer stabilisierten Ingwer-Extraktzubereitung dargestellt:

**Tabelle 6: Prüfung der thermischen Stabilität über 24 h (unbehandelter vs. stabilisierter Extrakt)**

| Temperaturen | Extr. Zingiberis e rhiz. spir. spiss. Nativer Extrakt, nicht stabilisiert | Ingwer-Extraktzubereitung stabilisiert mit Kollidon 25 Nativer Extraktanteil 10,5 % (Angaben ber. auf nativen Extraktanteil) |
|---|---|---|
| | Summe 6-, 8- und 10-Gingerol | Summe 6-, 8- und 10-Gingerol |
| Raumtemperatur | 13,3% | 13,3% |
| 40°C | 13,2 % | 13,2 % |
| 50°C | 13,2 % | 13,2 % |
| 60°C | 12,3 % | 13,1 % |
| 70°C | 11,5% | 12,2 % |
| | Verhältnis 6-Gingerol / 6-Shogaol | Verhältnis 6-Gingerol / 6-Shogaol |
| Raumtemperatur | 1,7 : 1 | 1,7 : 1 |
| 40°C | 1,7 : 1 | 1,7 : 1 |
| 50°C | 1,6 : 1 | 1,6 : 1 |
| 60°C | 1,4 : 1 | 1,6 : 1 |
| 70°C | 1,2 : 1 | 1,4 : 1 |

Die Ergebnisse zeigen, daß die stabilisierte Extraktzubereitung auch bei erhöhten Temperaturen deutlich verbesserte Stabilitätseigenschaften gegenüber bisher üblichen, nicht stabilisierten Ingwerextrakten aufweist. Dies wird durch eine geringere Tendenz zum Abbau der Gingerole sowie durch eine geringere Abnahme des 6-Gingerol/6-Shogaol-Verhältnisses belegt. Temperaturen bis zu 40-50 °C erweisen sich hiernach als unproblematisch. Auch der gleichzeitig geprüfte, unstabilisierte Ingwer-Softextrakt hielt der Kurzzeitbelastung in diesem Temperaturbereich noch stand, so daß die maximale Temperaturbelastung für eine schonende Primärextraktion von ca. 45°C abgeleitet werden konnte. Bekannte Extraktionstemperaturen für Ingwerwurzeln reichen von Raumtemperatur bis 90 °C, wobei bis zu letzterem Wert keine Qualitätsbeeinträchtigungenfür den Extrakt beschrieben sind (Govindarajan, V.S.: Crit. Rev.Food Sci. Nutr. 17, 189-258, 1982). Die neu gewonnenen Erkenntnisse beweisen jedoch, daß die bislang industrieüblichen Extraktionsbedingungen von >50 °C demnach schon bei der Primärextraktion der Ingwerwurzel zu Verlusten an Scharfstoffen und somit zu einem pharmazeutisch minderwertigen Extrakt führen.

Wie die Versuchsergebnisse insgesamt zeigen, bleiben die erfindungsgemäßen Extraktzubereitungen über einen langen Zeitraum von bis zu 18 Monaten und mehr stabil. Sie sind überraschenderweise auch thermisch deutlich stabiler als unbehandelte Extrakte. Für eine schonende Prozeßführung bei der Herstellung von Ingwerextrakten konnte insbesondere für die Primärextraktion, die noch ohne den Zusatz eines stabilisierenden Hilfsstoffes abläuft, ein Temperaturgrenzwert von maximal 45°C ermittelt werden. Sie verläuft somit schonend als auch erschöpfend (hohe Ausbeute pharmazeutisch relevanter Inhaltsstoffe). Auch für den weiteren Prozeßablauf zur Herstellung der Ingwer-Extraktzubereitung ist es vorzuziehen, entsprechend der Erfindung Temperaturen von maximal 45°C anzuwenden, da hierdurch die Zersetzung bzw. die Abnahme der pharmazeutisch relevanten Substanzen verringert werden kann.

Eine ebenso temperaturschonende Verarbeitung stellt die Extraktion mit überkritischem Kohlendioxid dar. Eine Kombination eines ethanolisch/wäßrig hergestellten Primärextrakts (Dickextrakt) und dessen nachgeschalteter Extraktion mit überkritischem Kohlendioxid führt zu einem an Scharfstoffen und ätherischem Öl sehr hoch angereicherten Ingwerextrakt. Gleichzeitig werden nicht wirksamkeitsrelevante, co-extrahierte Extraktbestandteile weitestgehend entfernt und weiter abgereichert.

Die Ausbeute dieser gekoppelten Verfahrensschritte Primärextraktion und CO₂-Extrak tion liegt nur bei ca. 2-3% bezogen auf die eingesetzte Drogenmenge, entsprechend einem DEV nativ (Droge-Extrakt-Verhältnis) von ca. 33-50:1. Dagegen führt eine übliche, direkte CO₂-Extraktion von Rhizoma Zingiberis zu einer Ausbeute von ca. 4 %, entsprechend einem DEV nativ von ca. 25:1.

Mit diesem hochangereicherten Extrakt, der gegenüber einer Einfachextraktion mit organischen Lösemitteln und einer direkten CO₂-Extraktion einen pharmazeutisch vollkommen neuer Stoff darstellt, führt die Zugabe einer protonenabfangenden Substanz zu einer hochdosierten, stabilisierten Ingwer-Extraktzubereitung.

### Beispiel 3

Ingwer-Spissumextrakt, analog wie in Vergleichsbeispiel 1 beschrieben hergestellt, enthält 15,8 % Gesamtscharfstoffe und 15,1 % ätherisches Öl (DEV nativ 12:1). Hiervon werden 10.0 kg Spissumextrakt auf 10,0 kg Kieselgur aufgezogen und mit einem CO₂-Durchsat von 10 kg / kg Mischung extrahiert. Die wasserfreie Ausbeute an öligem Ingwer-Extrakt beträgt 34 % bezogen auf den Einsatzextrakt. Der resultierende Ingwer-Oleosum enthält 22,7 % Gesamtscharfstoffe und 39,0 % aetherisches Öl (DEV nativ 35:1).

15 g Ingwer-Oleosum werden mit Ethanol > 99 % auf 20 % Trockensubstanz verdünnt. Es wird 30 Minuten lang bis zum Erhalt einer homogenen Lösung geführt - Lösung 1. 85 g Kollidon 25 werden unter Rühren mit Ethanol > 99 % auf 10 % Trockensubstanz verdünnt. Es wird 30 Minuten lang intensiv gerührt - Lösung 2.

Beide Lösungen werden unter Rühren bei Raumtemperatur portionsweise vereinigt, wobei eine rotbraune, klare Lösung entsteht. Es wird 30 Minuten lang gerührt und am Rotationsverdampfer schonend bei maximal 45°C Wasserbadtemperatur eingeengt. Die erhaltene Ingwer-Extraktzubereitung wird lösungsmittelfrei eingedampft und im Trockenschrank bei 45°C unter Vakuum getrocknet. Man erhält ein hellgelbes, rieselfähiges Pulver mit ca. 3 % Scharfstoffen.

### Beispiel 4

Es wird eine stabilisierte Ingwerextraktzubereitung gemäß Beispiel 2, enthaltend 28 % nativen Ingwerextrakt, 63 % Kollidon 25 und 9 % hochdisperses Siliziumdioxid hergestellt. Diese erfindungsgemäß hergestellte, feste Form einer stabilisierten Ingwer-Extraktzubereitung wird in einem Dragee verpreßt.

### Rezeptur eines Ingwer-Drageekerns:

| | |
|---|---|
| Ingwer-Extraktzubereitung | 303,57 mg |
| Natriumdodecylsulfat | 8,80 mg |
| Maltodextrin | 79,23 mg |
| Siliziumdioxid | 8,80 mg |
| Cellulosepulver | 11,00 mg |
| Na-Carboxymethylstärke | 22,00 mg |
| Stearinsäure | 6,60 mg |
| Na-Crosscarmelose | 13,00 mg |

Tabelle 7 gibt Aufschluß über die Stabilität von Ingwer-Extraktzubereitungen und daraus hergestellten Drageekernen.

**Tabelle 7: Vergleichende Stabilität von Ingwer-Extraktzubereitungen und Drageekernen über 6 Monate bei 25°C. (In Klammern ist das jeweils vorliegende Verhältnis 6-Gingerol zu 6-Shogaol als Maß für die Stabilität angegeben.)**

| Probenbezeichnung | Analytische Parameter | Startwert | 6 Monate |
|---|---|---|---|
| Eingesetzte Ingwer- | Scharfstoffgehalt | 7,12 % | 7,18% |
| Extraktzubereitung | Verhältnis 6-Gingerol : 6-Shogaol | (6,9:1) | (7,0:1) |
| Ingwer-Dragees (67 % | Scharfstoffgehalt | 4,82 % | 4,74% |
| Ingwerzubereitung) | Verhältnis 6-Gingerol : 6-Shogaol | (6,9:1) | (6,9:1) |

Die erfindungsgemäße Extraktzubereitung liefert für Ingwer erstmals ein hochdosiertes, stabiles Phytopharmakon, das auch in der festen galenischen Form eine gleichbleibende therapeutische Qualität gewährleistet.

## Patentansprüche

1. Stabile Ingwer-Extraktzubereitung, **gekennzeichnet durch** einen Gehalt eines Ingwerextraktes und mindestens eines stabilisierenden Hilfsstoffes aus der Gruppe der Polyvinylpyrrolidone und **dadurch**, daß es sich um eine trockene Extraktzubereitung handelt.

2. Stabile Ingwer-Extraktzubereitung nach Anspruch 1, **dadurch gekennzeichnet daß** der Scharfstoffgehalt und das Verhältnis von [6]-Gingerol zu [6]-Shogaol stabil bleiben.

3. Stabile Ingwer-Extraktzubereitung nach einem der Ansprüche 1 bis 2, **dadurch gekennzeichnet, daß** das Verhältnis von Hilfsstoff zu nativem Extrakt größer als 1:1 1 ist.

4. Stabile Ingwer-Extraktzubereitung nach Anspruch 3, bei der das Verhältnis Hilfsstoff zu nativem Extrakt 1,5:1 bis 9:1, vorzugsweise jedoch 3:1, beträgt.

5. Stabile Ingwerextraktzubereitung nach einem der Ansprüche 1 bis4, enthaltend
- mind. 1 % Scharfstoffe, bevorzugt 3-12 % Scharfstoffe
- ein Verhältnis von [6]-Gingerol zu [6]-Shogaol von mindestens 2:1, bevorzugt zwischen 3:1 und 8:1
- mind. 1% ätherisches Öl, bevorzugt 5-15% [V/m] sowie
- aflatoxinarm entsprechend den Vorgaben der gültigen Aflatoxin-Verbotsordnung.

6. Verfahren zur Herstellung einer stabilen Ingwer-Extraktzubereitung, nach einem der Ansprüche 1 bis 5, **dadurch gekennzeichnet, daß** die Verfahrensschritte bei einer Temperatur von maximal 45°C durchgeführt werden.

7. Verfahren nach Anspruch 6, **dadurch gekennzeichnet, daß** die Verfahrensschritte bevorzugt bei einer Temperatur zwischen 35 und 40°C durchgeführt werden.

8. Verfahren zur Herstellung einer stabilisierten Ingwer-Extraktzubereitung nach einem der Ansprüche 6 bis 7, **dadurch gekennzeichnet, daß** bei der Herstellung mindestens ein stabilisierender Hilfsstoff aus der Gruppe der Polyvinylpyrrolidone verwendet wird.

9. Verfahren zur Herstellung einer stabilisierten Ingwer-Extraktzubereitung nach einem der Ansprüche 6 bis 8, **dadurch gekennzeichnet, daß** die zur Extraktion des Ingwerwurzelstockes eingesetzten Auszugsmittel aus der Gruppe der Alkohole mit C1-C4 Kohlenstoffatomen, aus der Gruppe der aliphatischen Ketone mit C1-C5 Kohlenstoffatomen, aus der Gruppe aliphatischer Kohlenwasserstoffe, aus der Gruppe wässrig-alkoholischer Lösungsmittelgemische von 1-99 % [V/V], aus der Gruppe von Lösungsmittelgemischen aus Wasser und Ketonen von 1-99 % [V/V] stammen bzw. reines Wasser oder ein überkritisches Gas wie Kohlendioxid sind.

10. Verfahren nach einem der Ansprüche 6 bis 9, umfassend die Schritte:
- schonende Primärextraktion der zerkleinerten Ingwerwurzeln,
- schonende weitere Konzentrierung, insbesondere durch Extraktion mit überkritischem Kohlendioxid,
- Zugabe mindestens einer Lösung eines stabilisierenden Hilfsstoffes zu der flüssigen Extraktlösung mit 20% Trockensubstanz,
- abschließende schonende Eindampfung und Trocknung der so erhaltenen Extraktzubereitung.

11. Galenische Zubereitung enthaltend eine stabilisierte Ingwer-Extraktzubereitung nach einem der Ansprüche 1-5 oder erhältlich nach einem der Ansprüche 6 bis 10 sowie weitere handelsübliche Hilfsstoffe wie Siliziumdioxide, Maltodextrine, Magnesiumstearate, Cellulosen oder Carboxymethylstärken.

12. Galenische Zubereitung nach Anspruch 11, enthalten in jeglicher Form von Kapseln, Tabletten und Dragees.

13. Verwendung einer stabilisierten Ingwer-Extraktzubereitung nach einem der Ansprüche 1 bis 5, einer galenischen Zubereitung nach Anspruch 11 bis 12 oder erhältlich nach einem der Ansprüche 6 bis 10 zur Herstellung eines Arzneimittels zur Behandlung von dyspeptischen Beschwerden, von Symptomen der Reisekrankheit, eines Antiemetikums, eines Antidiabetikums, eines Analgetikums, eines Arzneimittels für schwangerschaftsbedingte oder chemotherapeutisch induziertes Erbrechen, zur Behandlung von Erkrankungen des rheumatischen Formenkreises oder eines Nahrungsergänzungsmittels.

## Claims

1. Stable ginger extract preparation, **characterised by** a content of a ginger extract and at least one stabilising auxiliary from the group of polyvinylpyrrolidones and by being a dry extract preparation.

2. Stable ginger extract preparation according to claim 1, **characterised in that** the pungent material content and the ratio of [6]-gingerol to [6]-shogaol remain stable.

3. Stable ginger extract preparation according to one of claims 1 to 2, **characterised in that** the ratio of auxiliary to natural extract is greater than 1 :1.

4. Stable ginger extract preparation according to claim 3, in which the ratio auxiliary to natural extract is between 1.5:1 and 9:1, but preferably 3:1.

5. Stable ginger extract preparation according to one of claims 1 to 4, containing
- at least 1% pungent materials, preferably 3-12% pungent materials
- a ratio of [6]-gingerol to [6]-shogaol of at least 2:1, preferably between 3:1 and 8:1
- at least 1% essential oil, preferably 5-15% [V/m] and
- low in aflatoxin corresponding to the conditions of the current aflatoxin prohibition order.

6. Process for producing a stable ginger extract preparation according to one of claims 1 to 5, **characterised in that** the process steps are carried out at a temperature of 45°C maximum.

7. Process according to claim 6, **characterised in that** the process steps are preferably carried out at a temperature between 35 and 40°C.

8. Process for producing a stabilised ginger extract preparation according to one of claims 6 to 7, **characterised in that** during production, at least one stabilising auxiliary from the group of polyvinylpyrrolidones is used.

9. Process for producing a stabilised ginger extract preparation according to one of claims 6 to 8, **characterised in that** the extraction agents used for extraction of the ginger rootstock originate from the group of alcohols having C1-C4 carbon atoms, from the group of aliphatic ketones having C1-C5 carbon atoms, from the group of aliphatic hydrocarbons, from the group of aqueous-alcoholic solvent mixtures from 1-99% [V/V], from the group of solvent mixtures of water and ketones from 1-99% [V/V] or are pure water or a supercritical gas, such as carbon dioxide.

10. Process according to one of claims 6 to 9, comprising the steps:
- gentle primary extraction of the comminuted ginger roots,
- gentle further concentration, in particular by extraction using supercritical carbon dioxide,
- addition of at least one solution of a stabilising auxiliary to the liquid extract solution with 20% dry solids,
- final gentle evaporation and drying of the extract preparation thus obtained.

11. Galenic preparation containing a stabilised ginger extract preparation according to one of claims 1-5 or obtainable according to one of claims 6 to 10 and further commercial auxiliaries, such as silicon dioxides, maltodextrins, magnesium stearates, celluloses or carboxymethyl starches.

12. Galenic preparation according to claim 11, present in any form of capsules, tablets and coated tablets.

13. Use of a stabilised ginger extract preparation according to one of claims 1 to 5, of a galenic preparation according to claim 11 to 12 or obtainable according to one of claims 6 to 10 for preparation of a medicament for the treatment of dyspeptic complaints, of symptoms of travel sickness, an anti-emetic agent, an anti-diabetic agent, an analgesic agent, a medicament for pregnancy-related or chemotherapeutically induced vomiting, for the treatment of diseases of the rheumatic type or a nutritional supplement.

## Revendications

1. Préparation stable à base d'extrait de gingembre, **caractérisée en ce qu'**elle contient un extrait de gingembre et au moins un auxiliaire de stabilisation choisi dans le groupe des polyvinyle pyrrolidones et **en ce qu'**elle est une préparation à base d'extrait sec.

2. Préparation stable à base d'extrait de gingembre selon la revendication 1, **caractérisée en ce que** la teneur en substances épicées et le rapport [6]-gingérol [6]-shogaol restent stables.

3. Préparation stable à base d'extrait de gingembre selon l'une des revendications 1 à 2, **caractérisée en ce que** le rapport auxiliaire extrait natif est supérieur à 1:1.

4. Préparation stable à base d'extrait de gingembre selon la revendication 3, dans laquelle le rapport auxiliaire extrait natif est de 1,5:1 à 9:1, et avantageusement de 3:1.

5. Préparation stable à base d'extrait de gingembre selon l'une des revendications 1 à 4, contenant
- au moins 1% de substances épicées, de préférence 3 à 12 % de substances épicées,
- un rapport [6]-gingérol [6]-shogaol d'au moins 2:1, de préférence entre 3:1 et 8:1,
- au moins 1% d'huile essentielle, de préférence 5 à 15% [V/m] ainsi que
- une très faible teneur en aflatoxines correspondant aux directives de la réglementation actuelle sur les aflatoxines.

6. Procédé de fabrication d'une préparation stable à base d'extrait de gingembre, selon l'une des revendications 1 à 5, **caractérisé en ce que** les étapes du procédé sont mises en oeuvre à une température de 45°C maximum.

7. Procédé selon la revendication 6, **caractérisé en ce que** les étapes du procédé sont mises en oeuvre de préférence à une température comprise entre 35°C et 40°C.

8. Procédé de fabrication d'une préparation stabilisée à base d'extrait de gingembre selon l'une des revendications 6 à 7, **caractérisé en ce que**, lors de la fabrication, on utilise au moins un auxiliaire de stabilisation choisi dans le groupe des polyvinyle pyrrolidones.

9. Procédé de fabrication d'une préparation stabilisée à base d'extrait de gingembre selon l'une des revendications 6 à 8, **caractérisé en ce que** les agents d'extraction utilisés pour extraire le rhizome de gingembre sont choisis dans le groupe des alcools comportant 1 à 4 atomes de carbone, dans le groupe des cétones aliphatiques comportant 1 à 5 atomes de carbone, dans le groupe des hydrocarbures aliphatiques, dans le groupe des mélanges de solvants hydroalcooliques de 1 à 99% [V/V], dans le groupe des mélanges de solvants constitués d'eau et de cétones de 1 à 99% [V/V] ou sont de l'eau pure ou un gaz supercritique tel que le dioxyde de carbone.

10. Procédé selon l'une des revendications 6 à 9, comportant les étapes consistant à :
- effectuer une extraction primaire soigneuse des racines de gingembre pulvérisées,
- effectuer une concentration supplémentaire soigneuse, notamment par extraction avec du dioxyde de carbone supercritique,
- et ajouter au moins une solution d'auxiliaire de stabilisation à la solution d'extrait liquide contenant 20% de substance sèche,
- effectuer enfin une concentration par évaporation et un séchage soigneux de la préparation d'extrait ainsi obtenue.

11. Préparation galénique contenant une préparation stabilisée à base d'extrait de gingembre selon l'une des revendications 1 à 5 ou obtenue selon l'une des revendications 6 à 10 ainsi que d'autres auxiliaires du commerce tel que du dioxyde de silicium, de la maltodextrine, du stéarate de magnésium, des celluloses ou des carboxyméthylamidons.

12. Préparation galénique selon la revendication 11, contenue dans des capsules, des comprimés et des dragées de forme quelconque.

13. Utilisation d'une préparation stabilisée à base d'extrait de gingembre selon l'une des revendications 1 à 5, d'une préparation galénique selon les revendications 11 à 12 ou obtenue selon l'une des revendications 6 à 10 pour la fabrication d'un médicament destiné à traiter des douleurs dyspeptiques ou des symptômes du mal des transport, la fabrication d'un antiémétique, d'un antidiabétique, d'un antalgique ou d'un médicament destiné à traiter les vomissements dus à une grossesse ou induits par une chimiothérapie ou à traiter des maladies de type rhumatismal, ou la fabrication d'un complément alimentaire.
